# FASCICULE DE BREVET EUROPEEN

(11) **EP 2 063 877 B1**
(45) Date de publication et mention de la délivrance du brevet: **13.01.2010**
(21) Numéro de dépôt: 07803872.6
(22) Date de dépôt: 16.07.2007
(51) Int. Cl.: A61K 31/00, A61K 31/437, A61K 31/445, G01N 33/53, A61P 21/00

(54) **MEDICAMENTS COMPRENANT UN INHIBITEUR DES ALPHA-MANNOSIDASES DE CLASSE I POUR LE TRAITEMENT DES SARCOGLYCANOPATHIES**
ARZNEIMITTEL ZUR BEHANDLUNG VON SARKOGLYKANOPATHIEN
DRUGS COMPRISING AT INHIBITOR OF CLASS I ALPHA MANNOSIDASE FOR THE TREATMENT OF SARCOGLYCANOPATHIES

(30) Priorité: 18.07.2006 FR 0653020
(43) Date de publication de la demande: 03.06.2009
(73) Titulaire: GENETHON, 91000 Evry (FR); Centre National de la Recherche Scientifique, 75794 Paris (FR)
(72) Inventeur: RICHARD, Isabelle, 91100 Corbeil Essonnes (FR)
(74) Mandataire: Denjean, Eric
(86) Numéro de dépôt international: PCT/FR2007/001211
(87) Numéro de publication internationale: WO 2008/009802

(56) Documents cités:
- EP-A- 1 618 881
- ASSERETO STEFANIA ET AL: "Pharmacological rescue of the dystrophin-glycoprotein complex in Duchenne and Becker skeletal muscle explants by proteasome inhibitor treatment" AMERICAN JOURNAL OF PHYSIOLOGY - CELL PHYSIOLOGY, vol. 290, no. 2, février 2006 (2006-02), pages C577-C582, XP002420099 ISSN: 0363-6143
- BONUCCELLI GLORIA ET AL: "Proteasome inhibitor (MG-132) treatment of mdx mice rescues the expression and membrane localization of dystrophin and dystrophin-associated proteins." AMERICAN JOURNAL OF PATHOLOGY, vol. 163, no. 4, octobre 2003 (2003-10), pages 1663-1675, XP002420100 ISSN: 0002-9440
- MATHEWS KATHERINE D ET AL: "LIMB-GIRDLE MUSCULAR DYSTROPHY" CURRENT NEUROLOGY AND NEUROSCIENCE REPORTS, CURRENT SCIENCE, PHILADELPHIA, PA, US, vol. 3, no. 1, janvier 2003 (2003-01), pages 78-85, XP008074976 ISSN: 1534-6293
- KAR N C ET AL: "GLYCOSIDASES IN NORMAL AND DISEASED HUMAN MUSCLE" CLINICA CHIMICA ACTA, vol. 45, no. 3, 1973, pages 269-271, XP002474352 ISSN: 0009-8981
- KAWAI HISAOMI ET AL: "Lysosomal enzyme activities in skeletal muscle of patients with neuromuscular diseases" MUSCLE AND NERVE, vol. 18, no. 9, 1995, pages 1009-1015, XP002474353 ISSN: 0148-639X

### SEQUENCE LISTING

<110> GENETHON
   Centre National de la Recherche Scientifique (CNRS)
<120> MEDICAMENTS POUR LE TRAITEMENT DES SARCOGLYCANOPATHIES
<130> G143-B-23358 PCT
<150> FR 06/53020
   <151> 2006-07-18
<160> 8
<170> PatentIn version 3.1
<210> 1
   <211> 29
   <212> DNA
   <213> Artificial Sequence
<220>
   <223> H77C (1)
<400> 1
   gcccaggtgg ctgtgctaca cacagcgca 29
<210> 2
   <211> 29
   <212> DNA
   <213> Artificial Sequence
<220>
   <223> H77C (2)
<400> 2
   tgcgctgtgt gtagcacagc cacctgggc 29
<210> 3
   <211> 20
   <212> DNA
   <213> artificial sequence
<220>
   <223> a-sarcoQ5'
<400> 3
   tataaccctg gcttcctcta 20
<210> 4
   <211> 24
   <212> DNA
   <213> artificial sequence
<220>
   <223> testNeo1
<400> 4
   cgaattcgcc aatgacaaga cgct 24
<210> 5
   <211> 20
   <212> DNA
   <213> artificial sequence
<220>
   <223> a-sarcoQ3'
<400> 5
   tagtggctca tgcctttaat 20
<210> 6
   <211> 20
   <212> DNA
   <213> artificial sequence
<220>
   <223> KIgenoseq2.s
<400> 6
   tgtgtttggg acttatgggg 20
<210> 7
   <211> 20
   <212> DNA
   <213> artificial sequence
<220>
   <223> KIgenoseq2.as
<400> 7
   caatcagcag cagcagcctc 20
<210> 8
   <211> 28
   <212> DNA
   <213> artificial sequence
<220>
   <223> R77C
<400> 8
   gccccggtgg ctctgctaca cccagcgc 28

## Description

### DOMAINE TECHNIQUE

La présente invention concerne le traitement des sarcoglycanopathies.

Elle préconise l'utilisation d'inhibiteurs de la mannosidase I, comme médicaments pour le traitement de certaines formes de ces maladies.

### ETAT ANTERIEUR DE LA TECHNIQUE

Les sarcoglycanopathies sont des maladies musculaires du groupe des myopathies des ceintures ("Limb Girdle Muscular Dystrophy" ou LGMD) et se transmettent sur le mode autosomique récessif. Quatre formes ont été identifiées (LGMD 2C, LGMD 2D, LGMD 2E, LGMD 2F), dues respectivement à des défauts dans les gènes de la γ-, α-, p- et δ-sarcoglycanes (11, 17).

La LGMD2C est particulièrement répandue dans le bassin méditerranéen et chez les populations tziganes résidant en Europe. La LGMD2D a été identifiée à travers le monde chez des patients en Europe, Afrique, Japon et Brésil. La LGMD2E a été retrouvé particulièrement dans la communauté amish et au Maghreb. La LGMD2F n'a été jusqu'à présent décrite que dans 7 familles d'origine brésilienne, turque et italienne. Les sarcoglycanopathies se traduisent par un déficit progressif des muscles des ceintures pelviennes et scapulaires fréquemment associée à une hypertrophie des mollets. L'intellect est toujours conservé. Une atteinte cardiaque de type cardiomyopathie est parfois retrouvée dans les LGMD2C, LGMD2E et LGMD2F.

Au niveau histologique, les muscles présentent des zones de régénération et de dégénération, des infiltrats inflammatoires, de la fibrose et une variabilité de la taille des fibres. Les sarcoglycanopathies présentent une sévérité variable, même au sein des fratries. Dans les formes les plus graves, les patients perdent la marche avant 30 ans et ont une espérance de vie réduite.

Deux des sarcoglycanopathies présentent des mutations récurrentes. Pour la LGMD2C, la mutation de1521T est fréquemment retrouvée dans les populations maghrébines et la mutation C283Y dans les populations tziganes (4, 13).

La mutation la plus fréquente présente dans la LGMD2D correspond à une substitution de l'arginine en position 77 par une cystéine (R77C). Cette mutation est retrouvée chez un tiers des patients en Europe à l'exception de la Finlande où elle est retrouvée chez 100% des patients la plupart du temps sur les deux allèles (5, 9).

Les sarcoglycanes (SG) constituent un groupe de glycoprotéines intégrées à la membrane plasmique et participant au complexe associé à la dystrophine (12). Ce complexe participe à la liaison entre le cytosquelette d'actine et la matrice extracellulaire et par conséquent à la stabilité de la membrane de la fibre musculaire (10). Les sarcoglycanes sont composées d'un petit domaine intracellulaire localisé en C-terminal (α-sarcoglycane) ou en N-terminal γ-, β- et δ-sarcoglycanes), un domaine transmembranaire unique et un large domaine extracellulaire qui contient des sites de N-glycosylation.

Le complexe des sarcoglycanes se forme au cours du transit de ces protéines dans le réticulum sarcoplasmique et l'appareil de Golgi (6, 16). La β-sarcoglycane sert d'initiateur à la formation du complexe. Son association avec la β-sarcoglycane positionne le complexe dans la membrane. Enfin, l'incorporation de l'α-sarcoglycane et son interaction avec la γ-sarcoglycane achève l'assemblage. Des mutations dans n'importe laquelle des sarcoglycanes peuvent empêcher le complexe de se former, entraînant une déficience secondaire des autres sarcoglycanes.

Si, aujourd'hui, on sait diagnostiquer les sarcoglycanopathies, et même identifier la composante mutée responsable du phénotype (γ-, α-, β- ou δ-sarcoglycane), ainsi que la mutation en cause, les moyens pour prendre en charge cette maladie restent modestes.

A ce jour, aucun traitement spécifique n'est connu et la plupart des patients nécessitent une kinésithérapie pour prévenir l'aggravation des contractures. Cependant, plusieurs équipes ont rapportés des résultats positifs au niveau expérimental par des approches de transfert de gène à l'aide de vecteurs viraux ou de thérapie cellulaire dans des modèles animaux (1, 7, 14). Ainsi, le document WO 00/20582 préconise une thérapie par remplacement des gènes de sarcoglycanes défectueux, véhiculés notamment par des AAV.

Il a également été proposé, dans l'art antérieur, une approche générale d'inhibition du protéasome pour le traitement des pathologies musculaires, en particulier des dystrophies musculaires. Toutefois, cette stratégie peu spécifique semble peu efficace et pose des problèmes de tolérance.

Il existe donc un besoin crucial de développer de nouvelles thérapies, notamment médicamenteuses, permettant de traiter spécifiquement et efficacement les sarcoglycanopathies.

### OBJET DE L'INVENTION

La présente invention repose sur la mise en évidence par les inventeurs que :
- certaines mutations dans les sarcoglycanes, associées à une sarcoglycanopathie, donnent lieu à des glycoprotéines présentant un mauvais repliement ;
- ces protéines "misfoldées" sont prises en charge par la voie de dégradation protéolytique des glycoprotéines associée au réticulum endoplasmique ;
- des inhibiteurs de cette voie bloquent la dégradation des sarcoglycanes mutées ;
- lorsqu'elles sont non dégradées (en présence d'inhibiteurs), les sarcoglycanes mutées peuvent être correctement transloquées à la membrane plasmique et intégrées au complexe des sarcoglycanes, ce qui se traduit par la restauration d'un phénotype normal.

Pour rappel, les glycosidases du réticulum endoplasmique (RE) jouent un rôle dans le contrôle de la qualité de la production des glycoprotéines. Elles assurent que seules celles , correctement repliées sont transportées vers leur destination finale. En particulier, le clivage des résidus mannose dans le RE par les α-mannosidases agit comme un signal pour diriger les glycoprotéines mal repliées vers une dégradation par le protéasome.

Les α-mannosidases sont classées en deux groupes. Celles de classe I (mannosidase I) sont inhibées par la 1-déoxymannojirimycin et la kifunensine, alors que celles de la classe II sont spécifiquement inhibées par la swainsonine. Le protéasome, quant à lui, est un complexe protéique impliqué dans la dégradation des protéines intracellulaires dans le cytosol et peut être inhibé par exemple par le MG132 ou le bortézomib.

A priori, tout inhibiteur de cette voie de dégradation (ERAD en anglais pour "Endoplasmic reticulum-associated dégradation") peut être utilisé. Toutefois, la présente invention s'est focalisée sur les inhibiteurs de la mannosidase 1 qui s'avèrent d'une grande sélectivité dans leur mode d'action et d'une efficacité remarquable pour le traitement des sarcoglycanopathies. Cliniquement, ceci se traduit notamment par des effets secondaires potentiellement moindres.

Dès lors, l'invention concerne l'utilisation d'inhibiteurs des α-mannosidases de classe I pour la préparation d'un médicament destiné au traitement des sarcoglycanopathies.

Il apparaît clairement, dans le contexte de l'invention, qu'on vise l'utilisation d'au moins un inhibiteur de la mannosidase I. Bien sûr, il n'est pas exclu d'utiliser un « cocktail » d'inhibiteurs, à savoir plusieurs inhibiteurs de nature différente, ayant des activités inhibitrices complémentaires, notamment en terme d'activité/sélectivité.

Sont tout particulièrement préférées la kifunensine (CAS 109944-15-2) et la 1-Deoxymannojirimycine (CAS 84444-90-6), molécules connues pour être des inhibiteurs de la mannosidase I.

Toutefois, l'invention ne saurait se limiter à ces molécules. En effet, toute molécule candidate peut être testée pour son activité inhibitrice de la mannosidase I, grâce à la mise en oeuvre d'un test enzymatique simple.

Ainsi, dans la mesure où il est recherché des solutions thérapeutiques pour les sarcoglycanopathies humaines, l'enzyme testée est avantageusement une alpha-mannosidase de classe I d'origine humaine, notamment celle référencée dans les bases de données sous le numéro (Accession Number) : NP_005898 (mannosidase, alpha, class 1A, member 1 [Homo sapiens]).

Un test enzymatique adapté pour tester de tels inhibiteurs est par exemple décrit dans la publication J Biol Chem. 2004 Oct 8;279(41):42638-47. Epub 2004 Jul 22. "The twisted abdomen phenotype of Drosophila POMTI and POMT2 mutants coincides with their heterophilic protein O-mannosyltransferase activity", Ichimiya T, Manya H, Ohmae Y, Yoshida H, Takahashi K, Ueda R, Endo T, Nishihara S.

Le mode d'administration, la dose administrée, ainsi que la fréquence d'administration sont déterminés au cas par cas, selon des protocoles classiques, connus de l'homme du métier. En effet, ces paramètres peuvent notamment dépendre de la mutation à traiter et de l'inhibiteur choisi.

La kifunensine peut être préférentiellement choisie. Elle s'avère d'une très grande spécificité et présente l'avantage d'être une molécule soluble dans l'eau, pouvant se prêter à une administration par voie orale.

En outre, ce traitement s'avère particulièrement approprié pour le traitement d'une sarcoglycanopathie liée à mutation R77C (Arg77Cys au niveau de la protéine ou 229C>T au niveau du gène) dans l'a-sarcoglycane humaine. Comme déjà dit, cette mutation est responsable de nombreux cas cliniques, notamment en Europe.

Toutefois, le Demandeur a montré que la 1-Deoxymannojirimycine pouvait aussi bien être utilisée, en lieu et place de la kifunensine.

De plus, il a été montré dans le cadre de l'invention que l'effet observé n'était pas lié à une mutation spécifique dans une sous-unité spécifique.

En effet, le même effet bénéfique a pu être observé dans les sous unités β et δ, plus précisément pour les mutations Q11E et E262K, respectivement, quel que soit l'inhibiteur en présence.

Ainsi, le présent traitement peut s'appliquer potentiellement à toutes les mutations aujourd'hui identifiées dans les quatre sarcoglycanes humaines. Toutefois, seules les mutations ponctuelles sont des candidats potentiels à ce traitement puisque dans le cas de codons stop ou de délétions, il est *a priori* exclu que la protéine tronquée, même si elle n'est pas dégradée, puisse être active.

Les principales mutations ponctuelles répertoriées à ce jour sont listées dans le tableau ci-dessous. Celui-ci ne se prétend toutefois pas comme étant exhaustif.

| α**-sarcoglycane** | | | β**-sarcoglycane** | | |
|---|---|---|---|---|---|
| Exon | Mutation dans gène | Mutation dans protéine | Exon | Mutation dans gène | Mutation dans protéin |
| | | | 01 | c 31C>G | p Gln11Glu |
| 02 | c.92T>C | p.Leu31pro | 03 | c.265G>A | p.Val89Met |
| 02 | c. 100C>T | p.Arg34Cys | 03 | c.271C>T | p.Arg91Cys |
| 02 | c. 101G>A | p.Arg34His | 03 | c.272G>C | p.Arg91Pro |
| 03 | c.184T>C | p.Tyr62His | 03 | c.274_275AT>TC | p.Ile92Ser |
| 03 | c.203G>A | p.Gly68Gln | 03 | c.275T>C | p.Ile92Thr |
| 03 | c.220C>T | p.Arg74Trp | 03 | c.299T>A | p.Met100Lys |
| **03** | **c.229C>T** | **p.Arg77Cys** | 03 | c.323T>G | p.Leu108A |
| 03 | c.266-267inv | p.Leu89Pro | 03 | c.341C>T | p.Ser114Phe |
| 03 | c.269A>G | p.Tyr90Cys | 03 | c.355A>T | p.IIe119Phe |
| 03 | c.271G>C | p.Gly91Arg | 03 | c.416G>A | p.Gly139Asp |
| 03 | c.278C>T | p.Ala93Val | 04 | c.452C>G | p.Thr151Arg |
| 03 | c.290A>G | p.Asp97Gly | 04 | c.499G>A | p.G1y167Ser |
| 03 | c.292C>T | p.Arg98Cys | 04 | c.538T>C | p.Phe180L |
| 03 | c.293G>A | p.Atg98His | 04 | c.544A>G | p.Thr182Ala |
| 03 | c.308T>C | p.Ile103Thr | 04 | c.551A>G | p.Tyr184Cys |
| 04 | c.329G>T | p.Arg110Leu | δ**-sarcoglycan**e | | |
| 04 | c.371T>C | p.Ile124Thr | 04 | c.212G>C | p.Arg71Thr |
| 05 | c.409G>A | p.G1u137Lys | 06 | c.451T>G | p.Ser151Ala |
| 05 | c.421C>A | p.Arg141Ser | 08 | c.593G>C | p.Arg198Pro |
| 05 | c.472C>T | p.Leu158Phe | 08 | c.631A>T | p.Asn211Tyr |
| 05 | c.5188T>C | p.Leu173Pro^{a} | 09 | **c.784C>A** | **p.Glu262Lys** |
| 05 | c.524T>C | p.Val175Ala^{a} | γ**-sarcoglycane** | | |
| 05 | c.541C>T | p.Arg181Gys | 03 | c.205G>C | p.Gly69Arg |
| 06 | c.586G>A | p.Val196Ile | 03 | c.206G>C | p.Gly69Asp |
| 06 | c.614C>A | p.Pro205His | 03 | c.269T>C | p.Leu90Ser |
| 06 | c.662G>A | p.Arg221His | 07 | c.581T>C | p.Leu194Ser |
| 06 | c.683C>A | p.Pro228Gln | 07 | c.629A>G | p.His210Arg |
| 06 | c.724G>T | p.Va1242Phe | 08 | c.787G>A | p.Glu263Lys |
| 06 | c.725T>C | p.Val242Ala | 08 | c.848G>A | p.Cys283Tyr |
| 06 | c.739G>A | p.Val247Met^{a} | | | |
| 07 | c.850C>T | p.Arg284Cys | | | |

Selon un second aspect, l'invention concerne également un procédé d'évaluation *in vitro* de l'efficacité d'un inhibiteur de la dégradation associée au réticulum endoplasmique (ERAD) sur la sarcoglycanopathie comprenant les étapes suivantes :
- cotransfection dans des cellules des quatre gènes codant les sarcoglycanes (γ-, α-, β- ou δ-), l'un des gènes étant porteur de la mutation testée ;
- incubation pendant plusieurs heures en présence de l'inhibiteur ;
- localisation du complexe des sarcoglycanes.

Avantageusement, il s'agit des sarcoglycanes humaines, portant des mutations ponctuelles.

Dans le cas où le complexe est localisé dans la membrane plasmique, il en est conclu que l'inhibiteur testé est efficace vis-à-vis de la mutation testée, et donc constitue une approche thérapeutique pour cette forme de sarcoglycanopathie.

La détection du complexe dans la membrane plasmique signifie :
- d'une part la prévention de la dégradation de la protéine mutée par l'inhibiteur ;
- et d'autre part une translocation correcte de la protéine mutée et son intégration dans le complexe au niveau de la membrane plasmique.

De manière avantageuse, le procédé est mis en oeuvre en parallèle :
- sur des cellules cotransfectés avec les gènes codant les quatre sarcoglycanes non mutées. Ceci constitue un témoin positif ;
- en réalisant une incubation en l'absence de l'inhibiteur testé. Ce témoin permet de conclure quant à l'effet éventuel de l'inhibiteur testé.

De manière préférée, la détection du complexe est réalisée par immunohistochimie, à l'aide d'anticorps dirigé contre au moins l'une des sarcoglycanes.

Alternativement, l'une des sarcoglycanes peut être marquée, notamment par fluorescence, puis détectée par microscopie ou par triage. Cette solution est avantageuse dans la mesure où l'étape de détection est moins lourde que la détection immunologique. Toutefois, il est important de vérifier que la protéine fusion (sarcoglycane + marquage) ne perturbe pas la formation du complexe.

Dans les deux cas, la détection (choix de l'anticorps ou choix de la sarcoglycane à fusionner) peut avoir pour cible la sarcoglycane mutée mais pas nécessairement.

A noter que ce procédé de criblage d'inhibiteurs peut être mis en oeuvre in vivo, notamment sur des souris porteuses, dans une des sarcoglycanes, de la mutation testée.

L'invention et les avantages qui en découlent ressortiront mieux des exemples de réalisation suivants, à l'appui des figures annexées.

L'invention est illustrée plus avant en rapport avec l'α-sarcoglycane portant la mutation R77C et en présence des inhibiteurs MG132 (inhibiteur du protéasome) et kifunensine (inhibiteur de la mannosidase I).
Figure 1 : Marquage immunohistochimique des α-, β-, γ-sarcoglycanes et de la dystrophine d'une biopsie musculaire d'un patient portant la mutation R77C de l'α-sarcoglycane.
Figure 2 : Marquage immunohistochimique des α-, β-, γ-sarcoglycanes et de la dystrophine chez la souris, au niveau de muscles normaux (WT), *Sgca*^{*77C*/}*^{77C}* et *Sgca-l-*
Figure 3 : Histologie des muscles *Sgca-*/*-,* WT et *Sgca*^{77c/77c} (Qua= quadriceps, Ga=gastrocnémius, Pso=psoas, Del= deltoide).
Figure 4 : Détection de cellules en nécrose par bleu Evans dans les muscles *Sgca-*/*-* et *Sgca*^{77C/77C}*,*
Figure 5 : Marquage immunohistochimique de l'α-sarcoglycane et la β-sarcoglycane de muscles Sgca-/- injectés par un AAV-humainSGCA77C. A gauche : α seule ; au centre : β seules à droite : α + β.
Figure 6 : Histologie des muscles Sgca-/- injectés par un AAV-Sgca77C (gauche: zone non transduite, droite : zone transduite).
Figure 7 : Marquage immunohistochimique de l'α-sarcoglycane de cellules quadri transfectées non perméabilisées. A gauche, quadritransfection par une α-sarcoglycane normale, à droite avec l'α-sarcoglycane mutée.
Figure 8 : Marquage immunohistochimique de l'α-sarcoglycane (à gauche) et de la calreticuline (au centre). Les deux marquages superposés sont montrés à droite.
Figure 9: Marquage immunohistochimique de l'α-sarcoglycane de cellules quadritransfectées avec une α-sarcoglycane mutée en présence de MG132.
Figure 10: Marquage immunohistochimique de l'α-sarcoglycane de cellules quadritransfectées avec une α-sarcoglycane mutée en présence de kifunensine.
Figure 11 : Marquage immunohistochimique de l'α-sarcoglycane montrant l'absence d'agrégats au niveau de muscle *Sgca-1-* injecté avec une α-sarcoglycane mutée et après traitement des souris par la kifunensine pendant une semaine.
Figure 12 : A/ Marquage immunohistochimique de l'α-sarcoglycane de cellules quadri transfectées avec les trois sarcoglycanes delta gamma beta et une α-sarcoglycane normale (à gauche), ou avec une α-sarcoglycane portant la mutation R77C en l'absence (au centre) ou en présence de 1-Deoxymannojirimycine (dMJ) 100 µM (à droite).
   B/ Marquage immunohistochimique de la δ-sarcoglycane de cellules quadri transfectées avec les trois sarcoglycanes alpha gamma beta et une δ-sarcoglycane portant la mutation E262K en l'absence ou en présence de 1-Deoxymannojirimycine (dMJ) 5 ou 100 µM.
   C/ Marquage immunohistochimique de la β-sarcoglycane de cellules quadri transfectées avec les trois sarcoglycanes delta gamma alpha et une β-sarcoglycane portant la mutation Q11E en l'absence ou en présence de 1-Deoxymannojirimycine (dMJ) 5, 50 ou 100 µM.

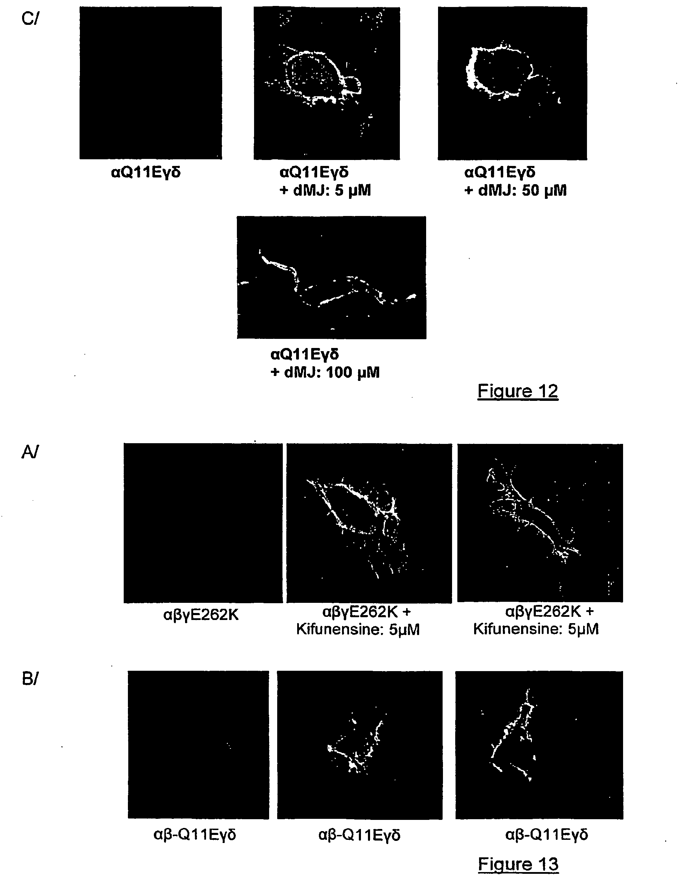

Figure 13 : A/ Marquage immunohistochimique de la δ-sarcoglycane de cellules quadri transfectées avec les trois sarcoglycanes alpha gamma beta et une δ-sarcoglycane portant la mutation E262K en l'absence ou en présence de kifunensine 5 µM.
   B/ Marquage immunohistochimique de la β-sarcoglycane de cellules quadri transfectées avec les trois sarcoglycanes delta gamma alpha et une β-sarcoglycane portant la mutation Q11E en l'absence ou en présence de kifunensine 5 µM.

### I) MATERIEL ET METHODES

### 1- Génération de la souris Sgca^{77c/77c}

Un fragment *BamH*I*-Sfi*I*.* de 1075 pb portant les exons 2 et 3 du gène Sgca est amplifié par PCR sur l'ADN d'un phage contenant le gène Sgca puis inséré dans le vecteur plasmidique pSP72 (Promega). La mutation H77C est introduite dans l'exon 3 par mutagénèse dirigée à l'aide des oligonucléotides suivants : 5'-GCCCAGGTGGCTGTGCTACACACAGCGCA-3' (SEQ ID 1) et 5'-TGCGCTGTGTGTAGCACAGCCACCTGGGC-3' (SEQ ID 2). La présence de la mutation ponctuelle est confirmée par séquençage. Un fragment 5' *Bgl*II*-Bam*HI d'environ 4 kb et un fragment 3' *Sfi*I*-Spe*I de 2981 pb sont clonés aux deux extrémités du fragment muté du vecteur pSP72. Une cassette *loxP-neo*^{r}*-loxP* du vecteur pGEM-neo^{r} est insérée au niveau d'un site *EcoR*V présent dans l'intron 3 et une cassette thymidine kinase (TK) est insérée en aval du fragment 3' pour réaliser le vecteur de recombinaison final.

Le vecteur de recombinaison (25 µg) est linéarisé par digestion *Sal*I et introduit dans des cellules ES 129Sv par électroporation. L'ADN des colonies résistantes au G418 est isolé et analysé par PCR ou Southern blot pour vérifier les événements de recombinaison. Deux clones recombines indépendants (IB4 et VIIICII) sont injectés dans des blastocystes C57B1/6 et des souris chimères sont produites. Les mâles chimériques sont croisés avec des femelles C57B1/6 pour produire des souris hétérozygotes. La cassette *neo*^{r} est éliminée par croisement avec la souche *deleter* (**15**). Les souris résultantes excisées de la cassette *neo*^{r} sont croisées entre elle pour produire des souris homozygotes mutantes. Le génotypage est réalisé par PCR sur de l'ADN de queue extrait par le kit Qiagen DNeasy Tissue Kit, en utilisant les oligonucléotides amont a-sarcoQ5': 5'-TATAACCCTGGCTTCCTCTA-3' (SEQ ID 3) et testNeol 5'-CGAATTCGCCAATGACAAGACGCT-3' (SEQ ID 4) et l'oligonucléotide aval a-sarcoQ3' 5'-TAGTGGCTCATGCCTTTAAT-3' (SEQ ID 5), produisant un fragment de 639 pb pour l'allèle mutant portent la cassette *neo^{r}* et un fragment de 484 pb pour l'allèle sauvage, dans les conditions de PCR suivantes : 94°C pendant 3 minutes, puis 30 cycles de 94°C pendant 30s, 61°C pendant 40s et 72°C pendant 1 minute, puis 3 minutes à 72°C. Après excision de la cassette *neo*^{r}*,* le génotype est réalisé avec l'oligonucléotide a-sarcoQ5' (SEQ ID 3) et l'oligonucléotide a-sarcoQ3' (SEQ ID 5) produisant un fragment de 575 pb pour l'allèle mutant.

Pour vérifier la présence de la mutation H77C dans le gène *Sgca* du modèle, une PCR avec les oligonucléotides KIgenoseq2.s: 5'-TGTGTTTGGGACTTATGGGG-3' (SEQ ID 6) et KIgenoseq2.as: 5'-CAATCAGCAGCAGCAGCCTC-3' (SEQ ID 7) est réalisée sur de l'ADN de queue, produisant un fragment de 659 pb qui est analysé par séquençage.

### 2 - Histologie et immunohistochimie

Les coupes (8 µm d'épaisseur) préparées à partir de muscle congelés sont colorées par hématoxyline et éosine (H&E). Les coupes correspondant aux souris injectées par bleu Evans sont révélées par excitation fluorescente à 633 nm.

Les coupes séchées, puis hydratées en PBS, ou les cellules fixées sont traitées 20 min avec 1% triton dilué en PBS, puis incubées 30 min à température ambiante (TA) en PBS contenant 15% de sérum de veau foetal. Des anticorps polyclonaux contre les sarcoglycanes (α-sarcoglycane : dilution 1/1000, dirigé contre les acides aminés 366-379 de l'α-sarcoglycane humaine ; β-sarcoglycane : dilution 1/20, NCL-b-SARC (Novocastra) ; γ-sarcoglycane : dilution 1/20, NCL-g-SARC (Novocastra) ; dystrophine : dilution 1/20, NCL-DYS2 (Novocastra) ; et calréticuline : dilution 1/70, ab4109 (Abcam)) sont déposés sur les coupes qui sont ensuite incubés 1 à 2 heures à TA puis lavées 3 fois en PBS. Les anticorps primaires sont révélés après incubation pendant 1 heure à TA avec un anticorps secondaire conjugué aux fluorochromes Alexa488 (A-11032, Molecular Probes) ou Alexa594 (A-11037, Molecular Probes), dilués au 1:1000 dans du PBS. Les coupes sont lavées trois fois en PBS, sont montées avec du fluoromount-G (SouthernBiotech 0100-01) puis examinées avec un microscope confocal (Leica). Les analyses d'immunohistochimie sur les biopsies humaines sont réalisées comme décrit dans Hackman *et al.* (9)*.*

### 3 - Constructions des plasmides et vecteurs AAV.

Les plasmides pAAV_C5-12_α-SG, pcDNA3_α-SG, pcDNA3_β-SG, pcDNA3_γ-SG et pcDNA3_δ-SG sont obtenus par PCR sur de l'ADNc de muscle squelettique et clones à l'aide du kit TOPO TA cloning kit (Invitrogen). Les sarcoglycanes sont ensuite sous-clonées dans le plasmide pcDNA3 (Invitrogen) ou pAAV_C5-12_MCS (3). Les plasmides pcDNA3_α-SG-R77C et pAAV_C5-12_α-SG-R77C sont obtenus à partir de pcDNA3-α-SG ou pAAV_C5-12_α-SG par mutagenèse dirigée en utilisant le kit Quickchange site-directed mutagenesis kit (Stratagene) à l'aide de l'oligonucléotide 5'-GCCCCGGTGGCTCTGCTACACCCAGCGC-3' (SEQ ID 8). Les constructions sont vérifiées par digestion enzymatique et séquençage.

Les préparations virales AAV 2/1 adenovirus-free sont obtenus en incorporant les génomes recombinant AAV2-ITRs dans des capsides AAV1 en utilisant un protocole de tritransfection (2). Les génomes viraux sont quantifiés par dot blot contre des dilutions sériées de plasmide standard.

### 4 - Cultures cellulaires

Les cellules NIH3T3 ou 911 sont cultivés en milieu modifié Dulbecco's D'Eagle, supplémenté avec de la glutamine, de la gentamicyne et 10% de sérum de veau foetal. Les cellules sont transfectées en utilisant 6 µl de Fugene (ROCHE) pour 1 µg de plasmide. Pour des boîtes 6 puits, 0,5 µg de chaque plasmide (α-SG ou α-SG-R77C et β-SG, γ-SG, δ-SG) est utilisé. Pour les traitements, les cellules sont incubées 43 heures après transfection avec l'inhibiteur de la mannosidase (5 µM de kifunensine, VWR) ou un inhibiteur du protéasome (5 µM de MG132 dissous dans du DMSO, Sigma) pendant 5 heures. Les cellules sont lavées en PBS et fixées 15 min à TA avec du formaldéhyde 3.7% en PBS puis rincées trois fois en PBS avant le marquage immunohistochimique.

### 5 - Expériences in vivo

Les souris *Sgca*^{77/77C} sont soumises à un exercice de course pendant 30 minutes sur un tapis roulant (Columbus treadmill instrument Exer 6M) pendant 3 jours consécutifs. Le tapis roulant est incliné selon une pente de 15 degré en descente et la vitesse fixée à 10 mètres par minute. A la fin des 3 jours, les souris sont injectés en intrapéritonéal par du bleu Evans (0,5 mg/g). Le jour suivant, les souris sont sacrifiées et les muscles deltoide, psoas, gastrocnemius, glutéus, extensor digitorum longus et quadriceps sont prélevés et rapidement congelés en isopentane refroidi à l'azote liquide.

Les préparations virales rAAV2/1 sont injectées (10¹⁰ viral génome (vg) dans 30 µl au total) dans le muscle tibialis anterior gauche de souris *Sgca-l-.* Aux jours 20, 22, 25 et 27 après injection, 10 µM de kifunensine ou 20 µM de MG132 sont injectés dans le muscle (soit 2 et 4 fois les concentrations utilisées *in vitro* pour tenir compte de la diffusion dans le muscle). Un jour avant sacrifice (jour 27), les souris sont injectées en intrapéritonéal par du bleu Evans. Les tibialis droit et gauche sont prélevés et rapidement congelés en isopentane refroidi à l'azote liquide.

### II) RESULTATS

### 1- La forme mutée R77C de l'α-sarcoglycane est absente de la membrane chez l'homme.

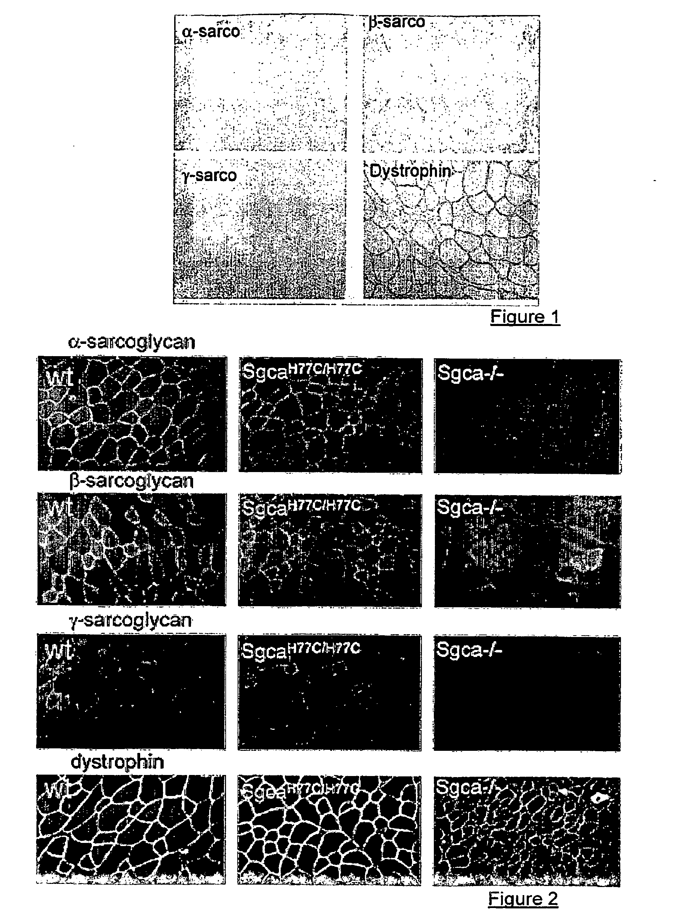

La présence de la mutation R77C sur l' α-sarcoglycane entraîne la déstabilisation du complexe des sarcoglycanes chez l'homme, comme montré par un marquage immunohistochimique à l'aide d'anticorps dirigée contre les différentes protéines du complexe (Figure 1).

### 2 - La présence d'une cystéine en position 77 chez la souris ne perturbe pas l'adressage à la membrane de l'α-sarcoglycane et n'entraîne pas de manifestation pathologique.

Afin d'étudier ce phénomène de déstabilisation, nous avons réalisé, par recombinaison homologue, un modèle animal *(Sgca*^{77C/77C}) portant une cystéine en position 77. II faut noter que l'espèce murine porte à cette position un résidu histidine au lieu d'un résidu arginine. L'analyse des muscles des souris ainsi obtenue par immunohistochimie à l'aide d'anticorps dirigé contre les différentes protéines du complexe DGC a montré que la présence de la mutation n'empêche pas l'adressage de l'α-sarcoglycane à la membrane ni la formation du complexe des sarcoglycanes (Figure 2). Des coupes de souris normales et déficientes en α-sarcoglycane *(Sgca*-/-*,* 8) ont été utilisées comme contrôle.

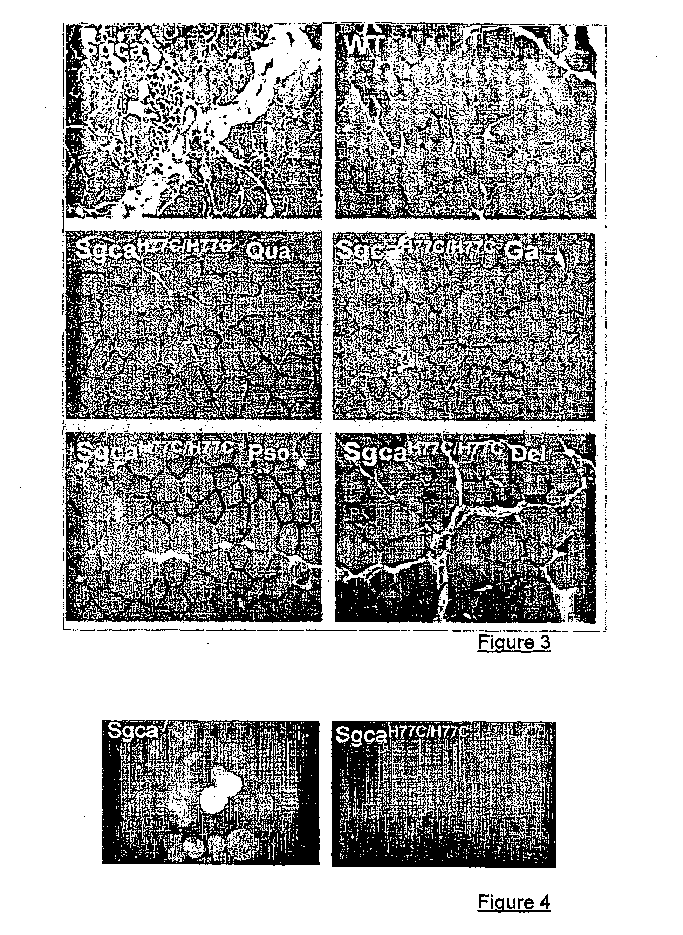

L'histologie des muscles deltoide, psoas, gastrocnemius et quadriceps issues de la souris *Sgca*^{77C/77C} âgée de 3 et 6 mois a été examinée par coloration à l'hématoxyline/éosine et comparée à celle de souris *Sgca*/*-.* Tandis que ces derniers présentent des signes dystrophiques sévères, aucune anomalie n'a pu être détectée au niveau des muscles *Sgca*^{77C/77C} (Figure 3).

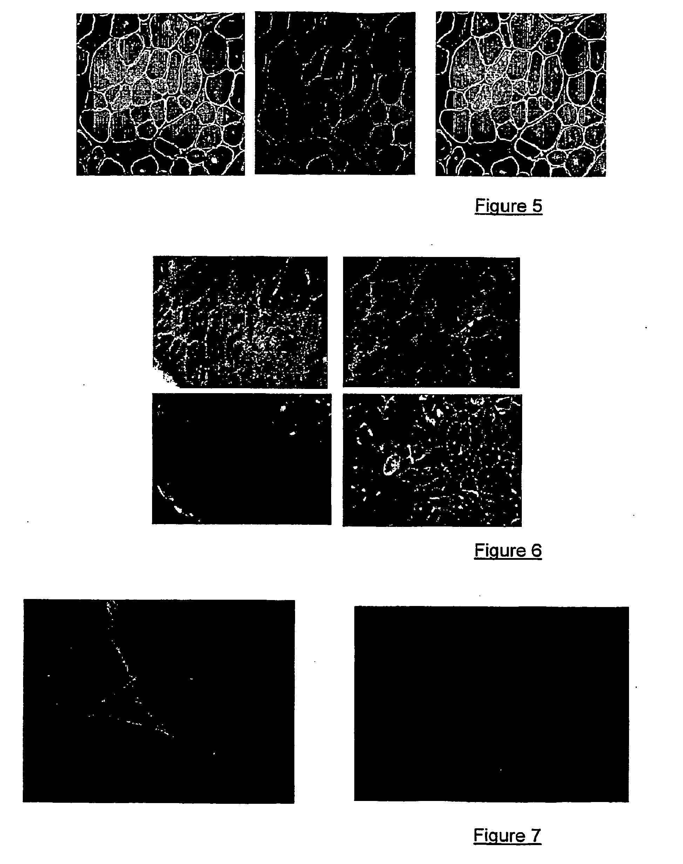

L'absence d'anomalies musculaires a été confirmée par une analyse fonctionnelle. Des souris *Sgca*^{77C/77C} ont été soumise à un exercice favorisant les contractions excentriques puis ont été injectées en intrapéritonéal par un colorant marquant spécifiquement les cellules en nécrose, le bleu Evans. Aucune pénétration de bleu Evans n'a pu être mis en évidence dans les muscles des souris *Sgca*^{77C/77C} (Figure 4). Afin de déterminer si les différences observées au niveau des conséquences de la présence d'un résidu cystéine en position 77 entre l'homme et la souris étaient liées à la nature de la protéine humaine, nous avons réalisé des expériences de transfert de gène dans le muscle de souris déficiente en α-sarcoglycane à l'aide d'un vecteur viral dérivé du virus associé à l'adénovirus (AAV) portant le gène humain de l'α-sarcoglycane mutée en position 77. L'analyse des muscles injectés a permis de montrer que la forme mutée se localise à la membrane bien qu'une partie soit retenue dans le réticulum, que le complexe sarcoglycane se forme et qu'elle corrige la pathologie (Figures 5 et 6). A noter à la figure 5 l'accumulation périnucléaire de l'α-sarcoglycane dans certaines cellules.

### 3 - La présence de la mutation entraîne une rétention de l'α-sarcoglycane et une dégradation par le protéasome.

Nous avons établi un modèle cellulaire reproduisant les phénomènes observés chez l'homme par quadruple transfection de plasmides codant pour les différentes sarcoglycanes. Dans ce modèle, le complexe est correctement formé à la membrane quand l'α-sarcoglycane normale est cotransfecté avec les trois autres sarcoglycanes, alors qu'il n'est pas détecté à la membrane lorsque l'α-sarcoglycane porte la mutation R77C. Ceci a pu être montré par un marquage immunohistochimique à l'aide d'un anticorps dirigé contre la partie extracellulàire de l'α-sarcoglycane sur des cellules non perméabilisées (Figure 7).

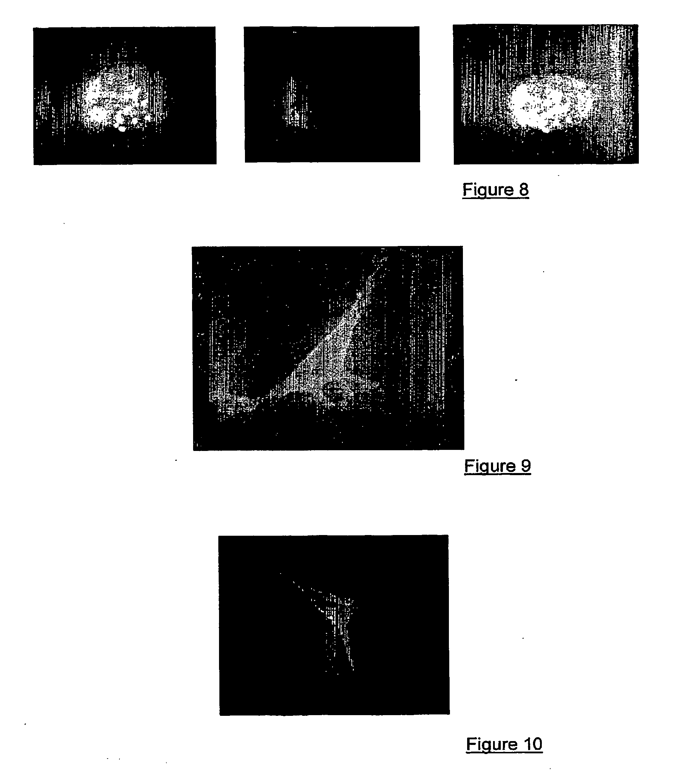

Un co-marquage de l'α-sarcoglycane avec un marqueur du réticulum endoplasmique (calreticulum) sur des cellules perméabilisées montre que la protéine mutée est retenue dans les voies de sécrétion (Figure 8).

Nous avons postulé que l'α-sarcoglycane mutée serait reconnue comme anormale par le système de contrôle-qualité des protéines au niveau du réticulum, et secondairement dégradée par le protéasome. Ceci a pu être confirmé par l'emploi de l'inhibiteur du protéasome MG132 qui permet de restaurer l'adressage à la membrane (Figure 9).

### 4 - L'adressage à la membrane est restauré par inhibition de la mannosidase I

Le système de contrôle-qualité du réticulum permet la dégradation de protéines mal repliées. L'enzyme mannosidase I joue un rôle important dans ce processus par l'intermédiaire d'une modification de chaînes d'oligosaccharides des protéines glycosylées, comme les sarcoglycanes. En fonction de cette donnée et de nos résultats, nous avons postulé que l'utilisation d'un inhibiteur de cette enzyme permettrait de restaurer l'adressage à la membrane de la forme mutée d'α-sarcoglycane. Cette hypothèse a été démontrée sur des cellules quadritransfectées avec une forme mutée d'α-sarcoglycane dans le modèle cellulaire traitée par la kifunensine (Figure 10).

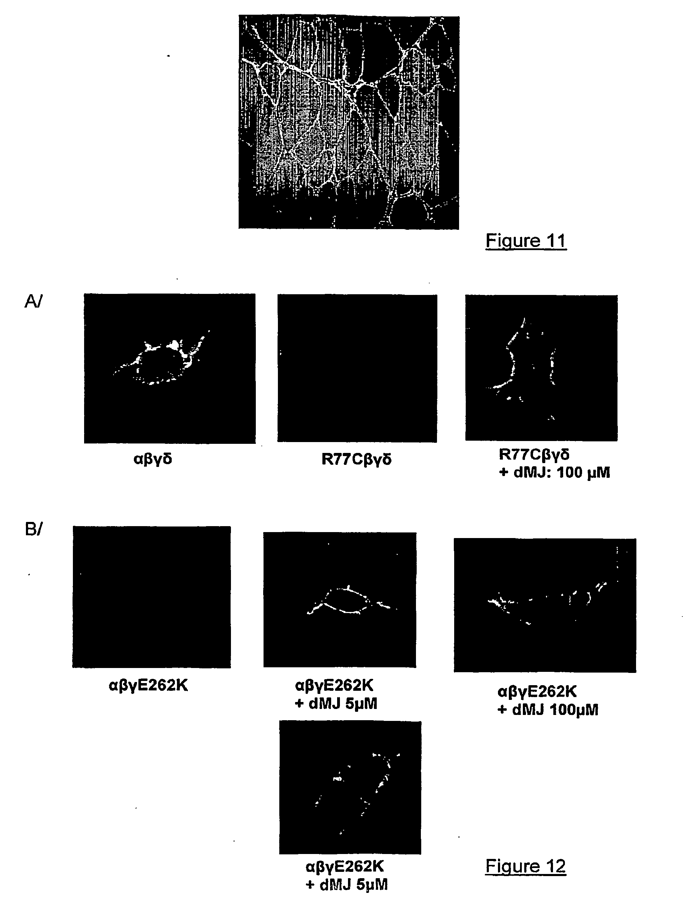

Les différences entre espèces humaine et murine observées précédemment ne nous permettent pas de disposer d'un modèle *in vivo* équivalent à l'homme. Cependant, une réduction de la rétention partielle de la forme mutée dans le réticulum après transfert de gène nous permettrait de suggérer que l'inhibition de la mannosidase I aurait un effet bénéfique sur l'adressage de l'α-sarcoglycane. Afin de déterminer si l'utilisation de l'inhibiteur pourrait être efficace *in vivo,* nous avons injecté ce produit en intramusculaire trois fois pendant une semaine dans les muscles de souris *Sgca-*/*-* ayant été préalablement injectée (15 jours avant) par le vecteur AAV-SGCA77C. Les observations des muscles injectées et traités montrent une absence quasi-totale des agrégats en particulier l'absence d'accumulation périnucléaire et donc valide l'hypothèse de travail (Figure 11).

Des résultats tout aussi convaincants ont été obtenus avec la kifunensine sur deux autres mutations ponctuelles affectant deux autres sous-unités du complexe des sarcoglycanes : mutation E262K portée par la sous-unité δ (Fig. 13A) et mutation Q11E portée par la sous-unité β (Fig. 13B).

### 5 - Restauration par la 1-Deoxymannojirimycine (dMJ)

Des expériences similaires à celles conduites en présence de kifunensine sur la mutation R77C de l'α-sarcoglycane ont été réalisées en présence de 1-Deoxymannojirimycine (dMJ), un autre inhibiteur connu des mannosidases de classe I. Les résultats apparaissent à la figure 12A et révèlent l'efficacité de cette substance pour rétablir l'adressage à la membrane de la forme mutée de l'α-sarcoglycane (mutation R77C).

En outre, des expériences similaires ont permis de valider l'hypothèse de travail sur d'autres mutations affectant d'autres sous-unités du complexe des sarcoglycanes : mutation E262K portée par la sous-unité δ (Fig. 12B) et mutation Q11E portée par la sous-unité β (Fig. 12C).

En conclusion, il a été montré que l'espèce murine se différencie de l'espèce humaine en ce qui concerne la prise en charge de la forme d'α-sarcoglycane portant une cystéine en position 77 et que cette forme mutée, quand elle est correctement localisée, est fonctionnelle et corrige la pathologie liée à la déficience en sarcoglycane. Nous avons montré dans un modèle cellulaire que l'inhibition de la mannosidase I empêche la dégradation de la forme mutée et son adressage correct à la membrane. L'utilisation *in vivo* de ce produit semble également exercer le même effet.

### BIBLIOGRAPHIE

1 - Allamand, V., K.M. Donahue, V. Straub, R.L. Davisson, B.L. Davidson, and K.P. Campbell. 2000. Early adenovirus-mediated gene transfer effectively prevents muscular dystrophy in alpha-sarcoglycan-deficient mice. Gene Ther. 7:1385-91.
2 - Bartoli, M., J. Poupiot, A. Goyenvalle, N. Perez, L. Garcia, O. Danos, and I. Richard. 2006a. Non-invasive Monitoring of Therapeutic Gene Transfer in Animal Models of Muscular Dystrophies. Gene Therapy. 13:20-8.
3 - Bartoli, M., C. Roudaut, S. Martin, F. Fougerousse, L. Suel, J. Poupiot, E. Gicquel, F. Noulet, O. Danos, and I. Richard. 2006b. Safety and efficacy of AAV-mediated calpain 3 gene transfer in a mouse model of limb-girdle muscular dystrophy type 2A. Mol Ther. 13:250-9.
4 - Bonnemann, C.G., J. Wong, K.J. Jones, H.G. Lidov, C.A. Feener, F. Shapiro, B.T. Darras, L.M. Kunkel, and K.N. North. 2002. Primary gamma-sarcoglycanopathy (LGMD 2C): broadening of the mutational spectrum guided by the immunohistochemical profile. Neuromuscul Disord. 12:273-80.
5 - Carrie, A, F. Piccolo, F. Leturcq, C. de Toma, K. Azibi, C. Beldjord, J.M. Vallat, L. Merlini, T. Voit, C. Sewry, J.A. Urtizberea, N. Romero, F.M. Tome, M. Fardeau, Y. Sunada, K.P. Campbell, J.C. Kaplan, and M. Jeanpierre. 1997. Mutational diversity and hot spots in the alpha-sarcoglycan gene in autosomal recessive muscular dystrophy (LGMD2D). J Med Genet. 34:470-5.
6 - Chan, Y.M., C.G. Bonnemann, H.G. Lidov, and L.M. Kunkel. 1998. Molecular organization of sarcoglycan complex in mouse myotubes in culture. J Cell Biol. 143:2033-44.
7 - Dressman, D., K. Araishi, M. Imamura, T. Sasaoka, L.A. Liu, E. Engvall, and E.P. Hoffman. 2002. Delivery of alpha- and beta-sarcoglycan by recombinant adeno-associated virus: efficient rescue of muscle, but differential toxicity. Hum Gene Ther 13:1631-46.
8 - Duclos, F., V. Straub, S.A. Moore, D.P. Venzke, R.F. Hrstka, R.H. Crosbie, M. Durbeej, C.S. Lebakken, A.J. Ettinger, J. van der Meulen, K.H. Holt, L.E. Lim, J.R. Sanes, B.L. Davidson, J.A. Faulkner, R. Williamson, and K.P. Campbell. 1998. Progressive muscular dystrophy in alpha-sarcoglycan-deficient mice. J Cell Biol. 142:1461-71.
9 - Hackman, P., V. Juvonen, J. Sarparanta, M. Penttinen, T. Aarimaa, M. Uusitalo, M. Auranen, H. Pihko, R. Alen, M. Junes, T. Lonnqvist, H. Kalimo, and B. Udd. 2004. Enrichment of the R77C alpha-sarcoglycan gene mutation in finnish LGMD2D patients. Muscle Nerve*.*
10 - Lapidos, K.A., R. Kakkar, and E.M. McNally. 2004. The dystrophin glycoprotein complex: signaling strength and integrity for the sarcolemma. Circ Res. 94:1023-31.
11 - Nigro, V. 2003. Molecular bases of autosomal recessive limb-girdle muscular dystrophies. Acta Myol. 22:35-42.
12 - Ozawa, E., Y. Mizuno, Y. Hagiwara, T. Sasaoka, and M. Yoshida. 2005. Molecular and cell biology of the sarcoglycan complex. Muscle Nerve*.*
13 - Piccolo, F., M. Jeanpierre, F. Leturcq, C. Dode, K. Azibi, A. Toutain, L. Merlini, L. Jarre, C. Navarro, R. Krishnamoorthy, F.M. Tome, J.A. Urtizberea, J.S. Beckmann, K.P. Campbell, and J.C. Kaplan. 1996. A founder mutation in the gamma-sarcoglycan gene of gypsies possibly predating their migration out of India. Hum Mol Genet. 5:2019-22.
14 - Sampaolesi, M., Y. Torrente, A. Innocenzi, R. Tonlorenzi, G. D'Antona, M.A. Pellegrino, R. Barresi, N. Bresolin, M.G. De Angelis, K.P. Campbell, R. Bottinelli, and G. Cossu. 2003. Cell therapy of alpha-sarcoglycan null dystrophic mice through intra-arterial delivery of mesoangioblasts. Science. 301:487-92.
15 - Schwenk, F., U. Baron, and K. Rajewsky. 1995. A cre-transgenic mouse strain for the ubiquitous deletion of loxP-flanked gene segments including deletion in germ cells. Nucleic Acids Res. 23:5080-1.
16 - Shi, W., Z. Chen, J. Schottenfeld, RC. Stahl, L.M. Kunkel, and Y.M. Chan. 2004. Specific assembly pathway of sarcoglycans is dependent on beta- and delta-sarcoglycan. Muscle Nerve. 29:409-19.
17 - Vainzof, M., M.R. Passos-Bueno, M. Canovas, E.S. Moreira, R.C. Pavanello, S.K. Marie, L.V. Anderson, C.G. Bonnemann, E.M. McNally, V. Nigro, L.M. Kunkel, and M. Zatz. 1996. The sarcoglycan complex in the six autosomal recessive limb-girdle muscular dystrophies. Hum Mol Genet. 5:1963-9.

## Revendications

1. Utilisation d'un inhibiteur des α-mannosidases de classe I pour la préparation d'un médicament destiné au traitement des sarcoglycanopathies.

2. Utilisation d'un inhibiteur selon la revendication 1, ***caractérisée* en ce que** l'inhibiteur est une molécule possédant une activité inhibitrice des α-mannosidases de classe I.

3. Utilisation d'un inhibiteur selon la revendication 2, **caractérisé en ce que** le composé est la kifunensine ou 1-Deoxymannojirimycine, avantageusement la kifunensine.

4. Utilisation d'un inhibiteur selon l'une des revendications 1 à 3, ***caractérisé* en ce que** la sarcoglycanopathie est liée à la mutation R77C dans l'α-sarcoglycane humaine.

5. Utilisation d'un inhibiteur selon l'une des revendications 1 à 3, ***caractérisé* en ce que** la sarcoglycanopathie est liée à la mutation E262K dans la δ-sarcoglycane humaine.

6. Utilisation d'un inhibiteur selon l'une des revendications 1 à 3, ***caractérisé* en ce que** la sarcoglycanopathie est liée à la mutation Q11E dans la β-sarcoglycane humaine.

7. Procédé d'évaluation *in vitro* de l'efficacité d'un inhibiteurs des α-mannosidases de classe I sur les sarcoglycanopathies comprenant les étapes suivantes :
- cotransfection dans des cellules des quatre gènes codant les sarcoglycanes γ-, α-, β-ou δ-), l'un des gènes étant porteur d'une mutation ;
- incubation pendant plusieurs heures en présence d'un inhibiteur tel que défini à l'une des revendications 1 à 3 ;
- localisation du complexe des sarcoglycanes.

8. Procédé d'évaluation *in vitro* de l'efficacité d'un inhibiteur selon la revendication 7 ***caractérisé* en ce que** le procédé est mis en oeuvre en parallèle sur des cellules cotransfectées par les gènes non mutés codant les quatre sarcoglycanes.

9. Procédé d'évaluation *in vitro* de l'efficacité d'un inhibiteur selon la revendication 7 ou 8 ***caractérisé* en ce que** le procédé est mis en oeuvre en parallèle sur des cellules incubées en l'absence de l'inhibiteur.

10. Procédé d'évaluation *in vitro* de l'efficacité d'un inhibiteurs selon l'une des revendications 7 à 9 ***caractérisé* en ce que** la localisation du complexe est réalisée par marquage immunohistochimique.

## Claims

1. Use of a class I α-mannosidase inhibitor for the preparation of a medicinal product intended to treat sarcoglycanopathies.

2. Use of an inhibitor according to claim 1, ***characterised* in that** the inhibitor is a molecule that has an inhibitory activity versus the class I α mannosidases.

3. Use of an inhibitor according to claim 2, ***characterised* in that** the substance is kifunensin or 1-deoxymannojirimycin, to advantage kifunensin.

4. Use of an inhibitor according to one of claims 1 to 3, ***characterised* in that** the disease is a sarcoglycanopathy associated with the R77C mutation on human α-sarcoglycan.

5. Use of an inhibitor according to one of claims 1 to 3, ***characterised* in that** the disease is a sarcoglycanopathy associated with the E262K mutation on human δ-sarcoglycan.

6. Use of an inhibitor according to one of claims 1 to 3, ***characterised* in that** the disease is a sarcoglycanopathy associated with the Q11E mutation on human δ-sarcoglycan.

7. *In vitro* assay for assessing the efficacy of a class I α-mannosidase inhibitor on sarcoglycanopathies comprising the following steps:
- co-transfection of cells with the four sarcoglycan genes (γ-, α-, β- and δ-), of which one carries a mutation;
- incubation for several hours in the presence of an inhibitor according to one of claims 1 to 3;
- localisation of the sarcoglycan complex.

8. *In vitro* assay for assessing inhibitor efficacy according to claim 7 ***characterised* in that** the assay is carried out in parallel on cells co-transfected with the genes coding for the four wild-type sarcoglycans.

9. *In vitro* assay for assessing inhibitor efficacy according to one of claims 7 or 8 ***characterised* in that** the assay is carried out in parallel on cells incubated without the inhibitor.

10. *In vitro* assay for assessing inhibitor efficacy according to one of claims 7 to 9 ***characterised* by** the use of immunohistochemical labelling techniques for localising the complex.

## Patentansprüche

1. Verwendung eines Hemmstoffs der α-Mannosidasen der Klasse 1 zur Herstellung eines zur Behandlung von Sarkoglykanopathien bestimmten Medikaments.

2. Verwendung eines Hemmstoffs nach Anspruch 1, ***dadurch gekennzeichnet, dass*** der Hemmstoff ein Molekül ist, das eine Hemmwirkung der α-Mannosidasen der Klasse 1 besitzt.

3. Verwendung eines Hemmstoffs nach Anspruch 2, **dadurch gekennzeichnet, dass** die Verbindung Kifunensin oder 1-Deoxymannojirimycin, vorteilhafter Weise Kifunensin ist.

4. Verwendung eines Hemmstoffs nach einem der Ansprüche 1 bis 3, **dadurch gekennzeichnet, dass** die Sarkoglykanopathie mit der R77C-Mutation im humanen α-Sarkoglykan verbunden ist.

5. Verwendung eines Hemmstoffs nach einem der Ansprüche 1 bis 3, **dadurch gekennzeichnet, dass** die Sarkoglykanopathie mit der E262K-Mutation im humanen S-Sarkoglykan verbunden ist.

6. Verwendung eines Hemmstoffs nach einem der Ansprüche 1 bis 3, **dadurch gekennzeichnet, dass** die Sarkoglykanopathie mit der Q11E-Mutation im humanen β-Sarkoglykan verbunden ist.

7. Verfahren zur *in vitro*-Auswertung der Wirksamkeit eines Hemmstoffs der α-Mannosidasen der Klasse 1 in Bezug auf Sarkoglykanopathien, folgende Schritte umfassend:
- gleichzeitige Transfektion von vier Genen, die (γ-, α-, β- oder δ-) Sarkoglykane kodieren, in Zellen, wobei eines der Gene ein Träger einer Mutation ist;
- mehrstündige Inkubation im Beisein eines wie in einem der Ansprüche 1 bis 3 definierten Hemmstoffs;
- Lokalisierung des Komplexes der Sarkoglykane.

8. Verfahren nach Anspruch 7, zur *in vitro*-Auswertung der Wirksamkeit eines Hemmstoffs, ***dadurch gekennzeichnet, dass*** das Verfahren parallel an mit nicht mutierten Genen, welche die vier Sarkoglykane kodieren, gleichzeitig transfizierten Zellen durchgeführt wird.

9. Verfahren nach Anspruch 7 oder 8, zur *in vitro*-Auswertung der Wirksamkeit eines Hemmstoffs, ***dadurch gekennzeichnet, dass*** das Verfahren parallel an in Abwesenheit eines Hemmstoffs inkubierten Zellen durchgeführt wird.

10. Verfahren nach einem der Ansprüche 7 bis 9, zur *in vitro*-Auswertung der Wirksamkeit eines Hemmstoffs, ***dadurch gekennzeichnet, dass*** die Lokalisierung des Komplexes durch immunohistochemische Markierung erfolgt.
